# EUROPÄISCHE PATENTSCHRIFT

(11) **EP 0 311 658 B1**
(45) Veröffentlichungstag und Bekanntmachung des Hinweises auf die Patenterteilung: **24.08.1994**
(21) Anmeldenummer: 88903188.6
(22) Anmeldetag: 25.04.1988
(51) Int. Cl.: A61B 17/22, A61B 17/36

(54) **VORRICHTUNG ZUR LASERBEHANDLUNG VON GEWEBE**
DEVICE FOR TREATING TISSUES WITH LASER
DISPOSITIF DE TRAITEMENT DE TISSUS AU LASER

(30) Priorität: 25.04.1987 DE 3713970
(43) Veröffentlichungstag der Anmeldung: 19.04.1989
(73) Patentinhaber: MEDOLAS GESELLSCHAFT FÜR MEDIZINTECHNIK mbH, D-82166 Gräfelfing (DE)
(72) Erfinder: MEDOLAS GESELLSCHAFT FÜR MEDIZINTECHNIK mbH, D-82166 Gräfelfing (DE)
(74) Vertreter: Münich, Wilhelm, Dr.
(86) Internationale Anmeldenummer: DE8800247
(87) Internationale Veröffentlichungsnummer: WO8808279

(56) Entgegenhaltungen:
- EP-A- 0 194 856
- EP-A- 0 195 375
- WO-A-85/02532
- GB-A- 2 125 986

## Beschreibung

### Technisches Gebiet

Die Erfindung bezieht sich auf eine Vorrichtung zur Laserbehandlung von Gewebe und insbesondere zum Abtragen von Gewebe mit einem Laser, dessen Strahl auf den zu behandelnden Bereich des Gewebes gerichtet ist.

Derartige Vorrichtungen können beispielsweise zur Angioplastie und insbesondere zur Behandlung von verkalkten Arterien, zur Meniskusoperationen, zur Krebsbehandlung verwendet werden.

### Stand der Technik

Als Laser sind in der Vergangenheit meist Neodym-YAG-Laser verwendet worden, seit einiger Zeit werden für derartige Behandlungen jedoch verstärkt Excimer-Laser, beispielsweise Xenonchlorid-, Kryptonfluorid oder Argonfluorid-Laser in Betracht gezogen. Excimer-Laser haben im Vergleich zu anderen Lasern den Vorteil, daß ihre Strahlung das Gewebe nicht erwärmt, sondern die Molekülbindungen Zerstört, so daß der Behandlungserfolg nicht über die Erwärmung des Gewebes, sondern über ein schichtweises Abtragen des Gewebes erzielt wird. Durch jeden Laserimpuls werden typischerweise (natürlich abhängig von der Impulsenergie und der Fleckgröße) Gewebeschichten mit einer Dicke zwischen 1»m und 5»m abgetragen.

Unabhängig davon, welche Laserstrahlung verwendet wird, tritt jedoch bei Operationen an unzugänglichen Stellen, wie im Kniegelenk, in der Niere und in Arterien, das Problem auf, daß der Operateur visuell kaum in der Lage ist, zu erkennen, ob eine gewünschte Schicht abgetragen oder bereits Gewebe, das nicht beschädigt werden soll, bestrahlt wird: Beispielsweise ist es bei der Angioplastie nur mit großer Erfahrung möglich, zu unterscheiden, ob Calcit (erwünscht) abgetragen wird oder die Laserstrahlung bereits die Arterienwand durchschneidet. Eine Vorrichtung gemäß Oberbegriff des Anspruchs 1 ist aus EP-A-0 195 375 bekannt.

### Darstellung der Erfindung

Der Erfindung liegt die Aufgabe, eine Vorrichtung zur Laserbehandlung von Gewebe und insbesondere zum Abtragen von Gewebe mit einem Laser derart weiterzubilden, daß es möglich ist, die Beschaffung der Gewebeart zu erkennen, auf die der Laser gerichtet ist, ohne daß eine weitere Lichtquelle zur Fluoreszenzanregung verwendet wird.

Eine erfindungsgemäße Lösung dieser Aufgabe ist mit ihren Weiterbildungen in den Patentansprüchen gekennzeichnet.

Die Erfindung geht von der Erkenntnis aus, daß Laserstrahlung und insbesondere Laserstrahlung im UV-Bereich biologisches Gewebe zu Fluoreszenzstrahlung anregt, deren spektrale Verteilung von der Gewebeart abhängt und so eine Identifizierung der bestrahlten Gewebeart erlaubt: So unterscheidet sich die Fluoreszenzstrahlung von Calcit deutlich von der des Muskelgewebes von Arterien. Auch die Fluoreszenzstrahlung von Menikusgewebe ist deutlich von der des umgebenden Knochengewebes verschieden. Weiterhin unterscheidet sich die Fluoreszenzstrahlung von Krebsgewebe von der des umgebenden Gewebes oder die der Augenlinse von der des Linsensackes.

Deshalb ist erfindungsgemäß wenigstens ein Sensor vorgesehen, der die von dem zu behandelnden Bereich des Gewebes aufgrund der auftreffenden Laserstrahlung ausgehende Fluoreszenzstrahlung erfaßt. Eine Spektralanalyseeinheit ermittelt die Spektralverteilung der vom Sensor erfaßten Fluoreszenzstrahlung und ermöglicht so eine Identifizierung der bestrahlten Gewebeart.

Je nach den Unterschieden zwischen den Gewebeschichten, die behandelt und insbesondere abgetragen werden sollen, und den Schichten, die durch die Laserstrahlung nicht beschädigt werden sollen, kann es erforderlich sein, daß die Spektralanalyseeinheit die Spektralverteilung über einen größeren Spektralbereich erfaßt und auswertet; bei zu differenzierenden Gewebearten, die stark unterschiedlich sind, kann es dagegen ausreichend sein, wenn die Spektralanalyseelnheit lediglich die Wellenlänge ermittelt, bei der das Maximum der Spektralverteilung liegt.

In jedem Falle ist es durch die erfindungsgemäßen Maßnahmen möglich, die von der Laserstrahlung getroffene Gewebeart zu identifizieren.

Weiterbildungen der Erfindung sind in den Unteransprüchen gekennzeichnet.

Selbstverständlich ist es möglich, den Sensor derart anzuordnen, daß er die von dem von der Laserstrahlung getroffenen Gewebe ausgehende Fluoreszenzstrahlung direkt erfaßt.

Besonders vorteilhaft ist jedoch die im Anspruch 2 gekennzeichnete Weiterbildung, bei der eine Strahlführungseinheit, die den Laserstrahl auf den zu behandelnden Bereich des Gewebes richtet, auch die Fluoreszenzstrahlung auf den Sensor führt. Diese Ausbildung hat aufgrund der Umkehrbarkeit des Lichtweges den Vorteil, daß praktisch nur die aus dem von der Laserstrahlung getroffenen Bereich ausgehende Strahlung erfaßt wird, so daß die erfindungsgemäße Vorrichtung u.a. kaum anfällig auf Umgebungsstrahlung etc. ist.

Die im Anspruch 2 gekennzeichnete Weiterbildung ist darüberhinaus dann von Vorteil, wenn die wenigstens eine Lichtleitfaser aufweisende Strahlführungseinheit (Anspruch 3) in ein Endoskop integriert ist, da dann der Sensor außerhalb des Endoskops angeordnet werden kann (Anspruch 4).

Das Ergebnis der von der Spektralanalyseeinheit durchgeführten Gewebeidentifizierung kann beispielsweise dem Operateur angezeigt werden, der dann den Behandlungsvorgang abbricht, wenn der Laser auf nicht zu behandelndes Gewebe, beispielsweise die Arterienwand gerichtet ist.

Besonders vorteilhaft ist es jedoch gemäß Anspruch 5, wenn eine Steuereinheit vorgesehen ist, die die Laserbehandlung unterbricht, wenn die Spektralanalyseeinheit eine nicht abzutragende oder auch eine nicht identifizierbare Gewebeart aufgrund der Spektralanalyse der Fluoreszenzstrahlung erkennt.

Da die Laserstrahlung von Excimerlasern nicht über die vom Gewebe aufgenommene Wärme, sondern durch die Zerstörung von Molekülbindungen wirkt, tritt nach Beendigung des Laserimpulses keine "Nachwirkung" auf. Die erfindungsgemäße Weiterbildung ermöglicht damit ein "punktgenaues" Abtragen des Gewebes, da bereits nach 1 bis 2 »m festgestellt werden kann, daß beispielsweise nicht mehr Calcit, sondern das Muskelgewebe der Arterienwand bestrahlt wird.

Excimerlaser arbeiten typischerweise mit Laserimpulsen mit einer Dauer von 20ns. Die durch einen Excimerlaserimpuls hervorgerufene Fluoreszenzstrahlung hat dagegen eine Dauer von typischerweise 5»s und eine Spektralverteilung mit einem Schwerpunkt je nach Gewebeart zwischen ca 350nm und 450 nm. Deshalb ist es nicht nur möglich, in einem Spektralbereich zu messen, für den Sensoren, Spektralanalyseeinheiten und Lichtleitfasern handelsüblich zur Verfügung stehen, sondern die Spektralanalyse kann auch nach Beendigung des Laserimpulses erfolgen. Darüberhinaus ist es von Vorteil, daß bei Excimerlasern keine Erwärmung des Gewebes erfolgt, so daß keine "Wärmestrahlung" auftritt, die die Fluoreszenzstrahlung überlagern könnte.

### Kurze Beschreibung der Zeichnung

Die Erfindung wird nachstehend anhand eines Ausführungsbeispiels unter Bezugnahme auf die Zeichnung näher beschrieben, in der zeigen:
- Figur 1: ein Blockschaltbild einer erfindungsgemäßen Vorrichtung,
- Figur 2 bis 4: verschiedene Spektrogramme.

### Darstellung eines Ausführungsbeispiels

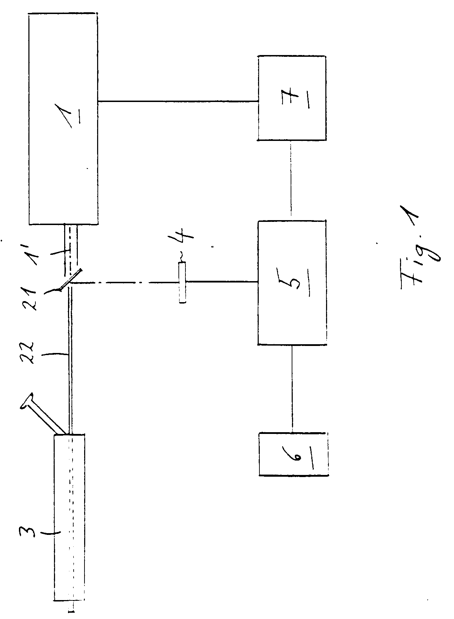

Figur 1 zeigt ein Blockschaltbild einer erfindungsgemäßen Vorrichtung, die sich beispielsweise zum Abtragen von Anlagerungen an Arterienwänden eignet. Die Vorrichtung weist einen Excimer-Laser (1) auf, wie er z.B. von der Firma Technolas hergestellt wird. Eine Strahlführungseinheit (2) leitet den Strahl (1') des Lasers (1) auf das zu behandelnde (nicht dargestellte) Gewebe. Die Strahlführungseinheit (2) besteht aus einer Auskoppeleinheit (21) und einer Lichtleitfaser (22), die teilweise in einem in die zu behandelnde Arterie einsetzbaren Instrument, beispielsweise einem nur schematisch dargestellten Endoskop (3) verläuft.

Die Auskoppeleinheit (21) besteht im wesentlichen aus einem teildurchlässigen und/oder mit unterschiedlicher Spektralcharakteristik versehenen Spiegel, der wenigstens einen Teil des durch die Lichtleitfaser (22) zurückgeleiteten Lichtes auf eine Sensoreinheit (4) leitet, die einen in der Spektralanalysetechnik übliche Aufbau haben kann.

Das Ausgangssignal der Sensoreinheit (4) liegt an einer an sich bekannten Spektralanalyseeinheit (5) an, die beispielsweise einen handelsüblichen Mikrocomputer aufweisen kann und deren Ausgangssignal auf einer Anzeigeeinheit (6) angezeigt wird. Ferner kann das Ausgangssignal der Spektralanalyseeinheit (5) dazu dienen, den Laser (1) anhand von bestimmten Kriterien über eine Steuereinheit (7) abzuschalten. Der Aufbau der Steuereinheit (7) kann ebenfalls aus handelsüblichen Komponenten erfolgen, so daß auf eine detaillierte Beschreibung verzichtet werden kann.

Die vorstehend schematisch beschriebene erfindungsgemäße Vorrichtung eignet sich damit zum einen zum Behandeln von Gewebe, beispielsweise zum Abtragen von sogenanntem Plaque und Calcit, d.h. zum Abtragen von Ablagerungen an der Arterieninnenwand.

Zum anderen wird die von dem behandelten Teil des Gewebes ausgehende Strahlung über die Lichtleitfaser (22) und den teildurchlässig bzw. spektralselektiv beschichteten Spiegel (21) von dem Sensor (4) erfaßt. Nach Beendigung eines Impulses des Excimer-Lasers (1) erfaßt der Sensor (4) ausschließlich die von dem behandelten Teil des Gewebes ausgehende Fluoreszenzstrahlung.

Erfindungsgemäß ist nun erkannt worden, daß anhand der Spektralverteilung bzw. anhand von Spektralmaxima der Fluoreszenzstrahlung eine Unterscheidung verschiedener Gewebearten möglich ist.

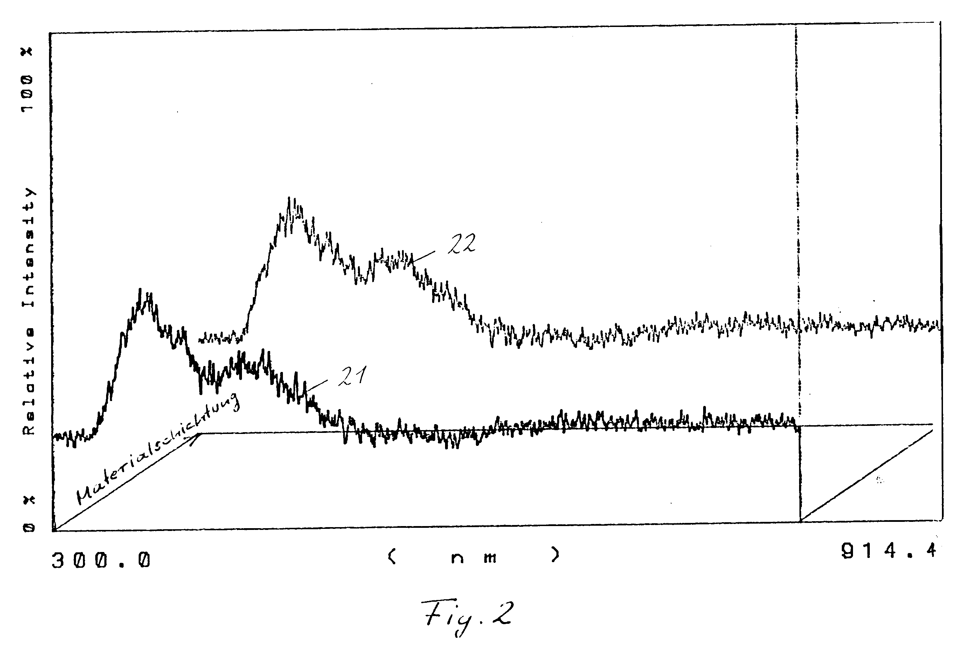

Figur 2 zeigt in einer "pseudo-dreidimensionalen" Darstellung Spektrogramme zu Beginn der Behandlung einer Arterienwand, auf der sich Plaque und calcifiziertes Gewebe (Gewebe mit Kalkeinlagerungen) auf dem gesunden Gewebe abgelagert haben. Dabei ist auf der horizontalen Achse die Wellenlänge der von dem Sensor (4) erfaßten Strahlung, auf der vertikalen Achse die Intensität der Strahlung und auf der "schräg nach hinten" gerichteten Achse die Materialschichtung aufgetragen. Figur 2 zeigt deutlich, daß die Spektrogramme (21) und (22) weitgehend übereinstimmen, daß also zunächst nur eine einheitliche Gewebeart, nämlich Plaque abgetragen wird.

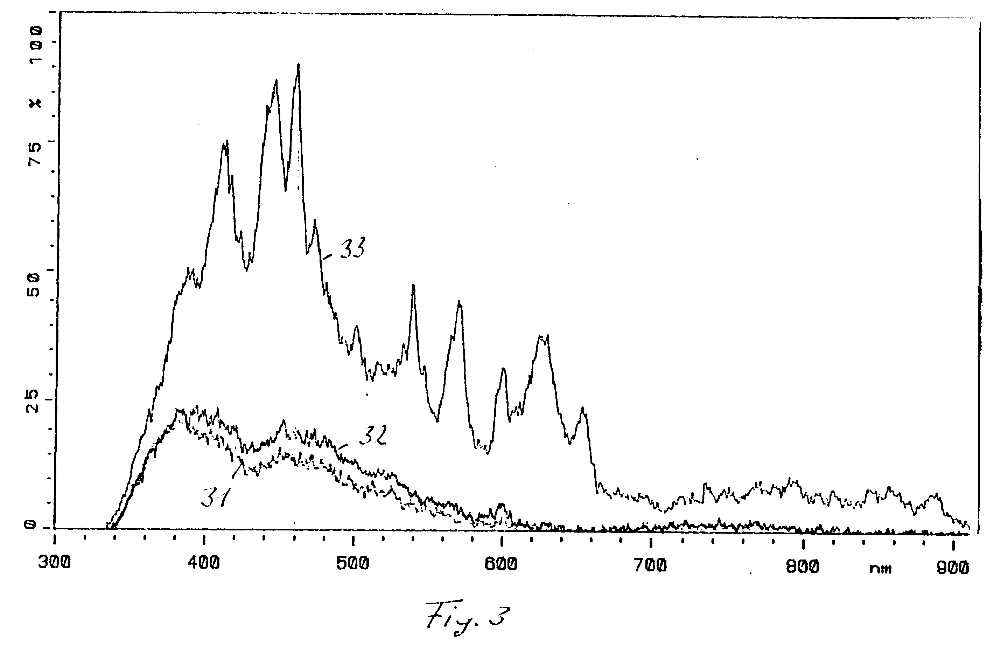

Figur 3 zeigt in einer zweidimensionalen Darstellung einen Vergleich der Spektrogramme von Plaque, an dem Übergang von Plaque zu calcifiziertem Gewebe und von calcifiziertem Gewebe.

Zunächst erhält man noch Kurven (Kurve 31), die den in Figur 2 gezeigten Kurven (21 und 22) gleichen und wie die in Figur 2 gezeigten Kurven zwei Maxima etwa bei 380 nm und 450 nm aufweisen. Am Übergang von Plaque zu calcifiziertem Gewebe (Kurve 32) wird zunächst das Maximum bei 450 nm, das zunächst deutlich kleiner als das Maximum bei 380nm gewesen ist, größer. Wenn das Plaque vollständig abgetragen worden ist und nur noch calcifiziertes Gewebe vorhanden ist, erhält man schließlich die Kurve (33), die sich durch eine Vielzahl von Maxima auszeichnet.

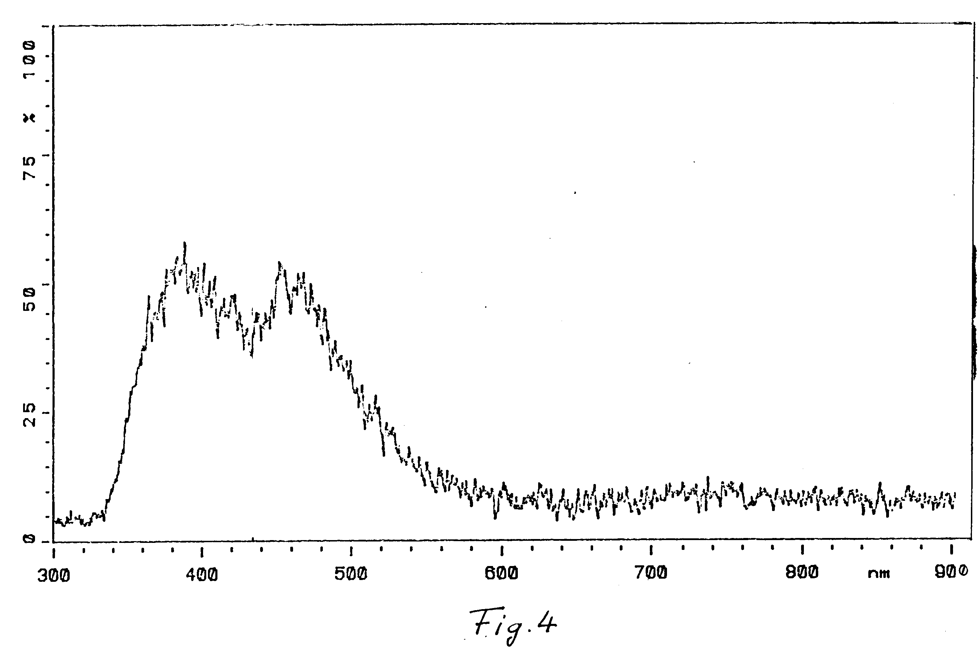

Figur 4 zeigt schließlich die bei normalem Arteriengewebe auftretende Fluoreszenzstrahlung. Diese Fluoreszenzstrahlung zeichnet sich durch zwei nahezu gleichstarke Maxima bei 390 und 480 nm sowie durch eine deutlich größere Intensität als bei Plaque aus.

Die in den Figuren 2 bis 4 gezeigten Spektrogramme zeigen deutlich, daß anhand des Spektralverlaufs der vom Sensor (4) erfaßten Fluoreszensstrahlung eine eindeutige Identifizierung der Gewebeart, auf die der Laserstrahl 1' auftrifft, möglich ist.

Insbesondere kann es unter Umständen ausreichend sein, nicht den gesamten Spektralverlauf zwischen 300 nm und 900 nm auszuwerten, sondern die auftretenden Spektrogramme anhand von charakteristischen Maxima zu bewerten.

Darüberhinaus hat die erfindungsgemäße Vorrichtung noch folgende Vorteile:
Da die Fluoreszenzstrahlung typischerweise eine Dauer von wenigstens 5»s hat, der Laserimpuls aber eine typische Dauer von 20 ns hat, ist eine leichte Trennung der reflektierten Strahlung von der Fluoreszenzstrahlung möglich.

Darüberhinaus liegt die Fluoreszenzstrahlung in einem deutlich längerwelligen Spektralbereich als die Strahlung des Excimer-Lasers, so daß auch "während der Dauer des Laserimpulses" jederzeit eine Trennung von reflektierter Strahlung und Fluoreszenzstrahlung mittels eines Spiegels mit geeigneter spektraler Charakteristik, beispielsweise eines Spiegels (4), der lediglich Strahlung mit längeren Wellenlängen als 300 nm reflektiert, möglich ist.

In jedem Falle ist die erfindungsgemäße Vorrichtung insbesondere bei Anwendungen für Operationen von Vorteil, bei denen ein Endoskop oder eine ähnliche Einrichtung Verwendung findet, wie bei der Angioplastie, bei Meniskusoperationen, bei Stein-Zertrümmerungen oder dgl.

Die Erfindung, die eine Identifizierung der mit Laserlicht bestrahlten Gewebeart erlaubt, kann aber natürlich auch bei Operationen "unter Sicht" verwendet werden, wie Hautkrebs-Operationen oder beim Herausschneiden der Augenlinse.

## Patentansprüche

1. Vorrichtung zur Laserbehandlung von Gewebe und insbesondere zum Abtragen von Geweben, mit
- einem Laser (1), dessen Strahl (1') auf den zu behandelnden Bereich des Gewebes gerichtet ist,
- wenigstens einem Sensor (4), der die von dem zu behandelnden Bereich des Gewebes ausgehende Fluoreszenzstrahlung erfaßt,
- einer Spektralanalyseeinheit (5), die zum Identifizieren der bestrahlten Gewebeart die Spektralverteilung der vom Sensor erfaßten Fluoreszenzstrahlung ermittelt und die Fluoreszenzstrahlung anhand von charakteristischen Maxima bewertet,
dadurch **gekennzeichnet**, daß der Laser ein im Impulsbetrieb arbeitender Excimer-Laser (1) ist, dessen Laserstrahl sowohl zur Behandlung des Gewebes als auch zur Erzeugung der Fluoreszenzstrahlung dient.

2. Vorrichtung nach Anspruch 1,
dadurch **gekennzeichnet**, daß eine Strahlführungseinheit (2), die den Laserstrahl (1') auf den zu behandelnden Bereich des Gewebes richtet, auch die vom Gewebe ausgehende Fluoreszenzstrahlung auf den Sensor (4) führt.

3. Vorrichtung nach Anspruch 2,
dadurch **gekennzeichnet**, daß die Strahlführungseinheit wenigstens eine Lichtleitfaser (22) aufweist.

4. Vorrichtung nach Anspruche 2 oder 3,
dadurch **gekennzeichnet**, daß wenigstens die Strahlführungseinheit (22) in ein Endoskop (3) integriert ist.

5. Vorrichtung nach einem der Ansprüche 1 bis 4,
dadurch **gekennzeichnet**, daß eine Steuereinheit (7) vorgesehen ist, die die Laserbehandlung unterbricht, wenn eine nicht abzutragende Gewebeart mittels der Fluoreszenzstrahlung identifiziert wird.

6. Vorrichtung nach einem der Ansprüche 1 bis 6,
dadurch **gekennzeichnet**, daß die Spektralanalyseeinheit (5) lediglich die vom bestrahlten Gewebe nach Beendigung des Laserimpulses ausgehende Laserstrahlung erfaßt.

7. Vorrichtung nach einem der Ansprüche 2 bis 7,
dadurch **gekennzeichnet**, daß ein Spiegel (21) vorgesehen ist, der die aus der Strahlführungseinheit (22) austretende vom Gewebe kommende Strahlung auf den Sensor (4) führt.

8. Vorrichtung nach Anspruch 8,
dadurch **gekennzeichnet**, daß der Spiegel (22) lediglich Licht mit einer größeren Wellenlänge als ca. 300 nm reflektiert.

## Claims

1. Device for treating tissues with laser, and in particular for removal of tissues, comprising
- a laser (1) whose beam (1') is directed onto the area of the tissue to be treated,
- at least one sensor (4) which detects the fluorescent radiation emanating from the tissue area to be treated,
- a spectral analyzer (5) which determines the spectral distribution of the fluorescent radiation detected by sensor, for identifying the irradiated type of tissue, and which assesses the fluorescent radiation by means of characteristic maximums,
**characterized** in that said laser is an excimer laser (1) operating in a pulsed mode, whose laser beam serves both to treat the tissue and to generate said fluorescent radiation.

2. Device according to Claim 1,
**characterized** in that a beam guiding means (2), which directs the laser beam onto the area of the tissue to be treated, also guides the fluorescent radiation emanating from the tissue onto said sensor (4).

3. Device according to Claim 2,
**characterized** in that said beam guiding means comprises at least one light-transmitting fibre (22).

4. Device according to Claims 2 or 3,
**characterized** in that at least said beam guide means (22) is integrated into an endoscope (3).

5. Device according to any of Claims 1 to 4,
**characterized** in that a control means (7) is provided for discontinuing the laser treatment if a type of tissue which is not to be removed is identified by means of said fluorescent radiation.

6. Device according to any of Claims 1 to 5,
**characterized** in that said spectral analyzer (5) merely detects the laser radiation emanating from the irradiated tissue upon termination of the laser pulse.

7. Apparatus according to any of Claims 2 to 6,
**characterized** in that a reflector (21) is provided which guides the radiation emanating from the tissue and discharged from said beam guide means (22) onto said sensor (4).

8. Apparatus according to Claim 7,
**characterized** in that said reflector (22) merely reflects light having a wavelength greater than 300 nm approximately.

## Revendications

1. Dispositif de traitement de tissus par laser, et en particulier à enlever de tissus, comprenant
- un laser (1) dont le faisceau (1') est dirigé sur la zone de tissu à traiter,
- au moins un détecteur (4) qui détecte le rayonnement fluorescent émis de la zone de tissu à traiter,
- un analyseur spectral (5) qui détecte la distribution spectrale du rayonnement fluorescent détecté par ledit détecteur, afin d'identifier le type de tissu irradié, et qui évalue le rayonnement fluorescent moyennant des maxima caractéristiques,
**caractérisés** en ce que ledit laser est un laser du type excimer fonctionnant en régime impulsionnel, dont le faisceau sert à traiter le tissu ainsi qu'à engendrer ledit rayonnement fluorescent

2. Dispositif selon la revendication 1,
**caractérisé** en ce'une unité de focalisation du faisceau électronique (2), qui focalise ledit faisceau laser sur la zone de tissu à traiter, focalise encore ledit rayonnement fluorescent émis du tissu sur ledit détecteur (4).

3. Dispositif selon la revendication 2,
**caractérisé** en ce que ladite unité de focalisation du faisceau comprend au moins une fibre optique (22).

4. Dispositif selon les revendications 2 ou 3,
**caractérisé** en ce qu'au moins ladite unité de focalisation du faisceau (22) est intégrée dans un endoscope (3).

5. Dispositif selon une quelconque des revendications 1 à 4,
**caractérisé** en ce qu'un organe de commande (7) est disposé qui arrête le traitement par laser si un type de tissu qu'on ne peut pas enlever est identifié moyennant ledit rayonnement fluorescent.

6. Dispositif selon une quelconque des revendications 1 à 5,
**caractérisé** en ce que ledit analyseur spectral (5) ne détecte que le rayonnement laser émis du tissu irradié après la fin de l'impulsion du laser.

7. Dispositif selon une quelconque des revendications 1 à 6,
**caractérisé** en ce qu'un miroir (21) est prévu pour focaliser la radiation qui sort de ladite unité de focalisation (22) et est émis par le tissu sur ledit détecteur (4).

8. Dispositif selon une quelconque des revendications 1 à 7,
**caractérisé** en ce que ledit (22) est prévu pour ne réfléchir que de lumière à une longueur d'onde dépassante 300 nm environ.
